# FASCICULE DE BREVET EUROPEEN

(11) **EP 3 840 787 B1**
(45) Date de publication et mention de la délivrance du brevet: **26.10.2022**
(21) Numéro de dépôt: 19795268.2
(22) Date de dépôt: 25.09.2019
(51) Int. Cl.: B01D 53/82, A61L 2/20, B01D 53/04

(54) **SYSTEME DE TRAITEMENT DE GAZ A DUREE DE VIE ACCRUE**
GASBEHANDLUNGSSYSTEM MIT ERHÖHTER LEBENSDAUER
GAS TREATMENT SYSTEM HAVING INCREASED SERVICE LIFE

(30) Priorité: 28.09.2018 FR 1859034
(43) Date de publication de la demande: 30.06.2021
(73) Titulaire: Commissariat à l'énergie atomique et aux énergies alternatives, 75015 Paris (FR)
(72) Inventeur: DELLEA, Olivier, 38054 GRENOBLE cedex 09 (FR); ROUSSEY, Arthur, 38054 Grenoble Cedex 09 (FR)
(74) Mandataire: Brevalex
(86) Numéro de dépôt international: PCT/FR2019/052258
(87) Numéro de publication internationale: WO 2020/065215

(56) Documents cités:
- EP-A1- 0 431 648
- CN-A- 107 469 562
- FR-A1- 2 972 932
- US-A1- 2004 140 194
- US-A1- 2008 210 084
- US-B1- 6 358 374

## Description

### DOMAINE TECHNIQUE

La présente invention concerne un système de traitement de gaz, tel qu'un système pour l'épuration de l'air intérieur de bâtiments, apte à fonctionner de manière alternée dans un mode de traitement et dans un mode de régénération, comprenant :
- une entrée et une sortie, et un chemin principal reliant l'entrée à la sortie et comprenant une portion de traitement, de manière à permettre la circulation d'un flux gazeux principal depuis l'entrée jusqu'à la sortie en passant par la portion de traitement, dans le mode de traitement ;
- au moins un chemin secondaire présentant deux extrémités opposées raccordées au chemin principal respectivement en amont et en aval de la portion de traitement de sorte que le chemin secondaire et au moins la portion de traitement du chemin principal forment une boucle de recirculation permettant une circulation en boucle d'un flux gazeux de recirculation dans la portion de traitement du chemin principal, dans le mode de régénération ;
- un ventilateur disposé dans la boucle de recirculation et configuré au moins pour propulser le flux gazeux de recirculation au sein de la boucle de recirculation dans un sens de circulation prédéterminé, dans le mode de régénération ;
- un premier dispositif d'adsorption disposé dans la portion de traitement du chemin principal, en aval du ventilateur eu égard au sens de circulation prédéterminé, et adapté pour adsorber des substances prédéfinies éventuellement présentes dans le flux gazeux principal, telles que des composés organiques volatils, dans le mode de traitement ;
- un générateur d'ozone disposé dans la portion de traitement du chemin principal, en aval du ventilateur eu égard au sens de circulation prédéterminé, et adapté pour générer de l'ozone dans le mode de régénération ; et
- un dispositif de commutation configuré pour maintenir l'entrée et la sortie ouvertes, empêcher la circulation du flux gazeux principal au travers du chemin secondaire ou de chaque chemin secondaire, et maintenir le générateur d'ozone à l'arrêt, dans le mode de traitement, et pour maintenir l'entrée et la sortie fermées, autoriser la circulation du flux gazeux de recirculation dans le chemin secondaire ou dans chaque chemin secondaire, et maintenir le générateur d'ozone en fonctionnement, dans le mode de régénération.

L'invention concerne également un procédé de traitement de gaz, en particulier un procédé d'épuration de l'air intérieur de bâtiments, au moyen d'un tel système.

### ÉTAT DE LA TECHNIQUE ANTÉRIEURE

Différentes technologies de traitement de gaz sont connues, en particulier en ce qui concerne le traitement de l'air intérieur des bâtiments. Celles-ci visent notamment à réduire les concentrations en composés organiques volatils (COVs).

Dans ce qui précède et ce qui suit, un « composé organique volatil » est défini conformément à la Directive 1999/13/CE du Conseil Européen du 11 mars 1999 en vertu de laquelle :
- un composé organique volatil est « *tout composé organique ayant une pression de vapeur de 0,01 kPa (soit 9,87.10⁻⁵ atm) ou plus à une température de 293,15 K (soit 20°C) ou ayant une volatilité correspondante dans les conditions d'utilisation particulières* » (cf. paragraphe 17 de l'article 2 de la Directive) ;
- un composé organique est « *tout composé comprenant au moins l'élément carbone et un ou plusieurs des éléments suivants : hydrogène, halogènes, oxygène, soufre, phosphore, silicium ou azote, à l'exception des oxydes de carbone et des carbonates et bicarbonates inorganiques* » (cf. paragraphe 16 de l'article 2 de la Directive).

Ainsi, sont notamment considérés comme des composés organiques volatils certains hydrocarbures acycliques, saturés ou insaturés, tels que l'éthane, le propane, le *n*-butane, le *n*-hexane, l'éthylène, le propylène, le 1,3-butadiène et l'acétylène, certains hydrocarbures cycliques non aromatiques, saturés ou insaturés, tels que le cyclopropane, le cyclopentane et le cyclohexane, certains hydrocarbures aromatiques tels que le benzène, le toluène, les xylènes et l'éthylbenzène, certains hydrocarbures halogénés tels que le dichlorométhane, le trichlorométhane, le chloroéthane, le trichloroéthylène et le tétrachloroéthylène, certains alcools tels que le méthanol, l'éthanol, le 1-propanol, le 2-propanol, l'éthylène glycol et le propylène glycol, certains aldéhydes tels que le formaldéhyde, l'acétaldéhyde, le propanal et le 2-propenal (ou acroléine), certaines cétones telles que l'acétone, la méthyléthylcétone, la 2-butanone et la méthylvinylcétone, certains esters tels que l'acétate de méthyle, l'acétate d'éthyle, l'acétate d'isopropyle et le butyrate d'isoamyle, certains éthers tels que l'éther diéthylique, le n-butyléther d'éthylène glycol (EGBE) et le 1,4-dioxane, certains acides tels que l'acide acétique et l'acide propanoïque, certaines amines telles que l'éthylamine, la diméthylamine, la triméthylamine, la diéthylamine et l'amylamine, certains amides tels que le diméthylformamide, les composés sulfurés tels que le mercaptan méthylique (ou méthanethiol) et le mercaptan éthylique (ou éthanethiol), et certains nitriles tels que l'acétonitrile et l'acrylonitrile.

Les technologies de traitement de gaz, en particulier les technologies de traitement de l'air intérieur de bâtiments, reposent sur des phénomènes physiques tels que :
- L'adsorption physique ou physisorption : le principe de l'adsorption repose sur la propriété qu'ont des solides (adsorbants) de fixer sur leur surface certains gaz (adsorbats). Les polluants sont adsorbés en général sur des charbons actifs ou des zéolithes (ces deux matériaux présentant l'avantage d'une surface spécifique importante et donc une grande capacité de stockage). Il s'agit d'une technologie efficace et peu coûteuse mais qui présente l'inconvénient de devoir changer très régulièrement les filtres pour ne pas les saturer et éviter la réémission des polluants.
- L'adsorption chimique ou chimisorption : le processus résulte d'une réaction chimique avec formation de liaisons covalentes ou ioniques entre les molécules d'adsorbat et la surface d'adsorbant. Le processus requiert dont les filtres soient régulièrement remplacés.
- L'ozonation : cette méthode consiste à faire passer l'air pollué dans un milieu à forte concentration d'ozone (généré par lampe UV ou plasma froid). C'est donc un procédé d'oxydation directe des polluants par l'ozone et ses radicaux dans le volume gazeux. Il est connu que ce procédé peut générer des produits polluants.
- La photolyse : l'air pollué passe dans un faisceau UV de haute énergie. Il est admis que le processus peut également générer des produits polluants. Par ailleurs, la lumière UV constitue un danger potentiel pour l'utilisateur, et ce procédé requiert une énergie importante.
- La photocatalyse : des radiations UV sont associées à un photocatalyseur (généralement du dioxyde de titane) pour générer des radicaux capables de dégrader les polluants tels que les COVs jusqu'à obtenir H20 ou C02 et tuer les espèces pathogènes. Les inconvénients sont la possibilité de réémettre des produits liés aux processus de dégradation, une faible efficacité et un taux de conversion faible.
- L'oxydation catalytique : des matériaux catalytiques sont utilisés pour dégrader les polluants tels que les COVs. Pour être efficace, ce procédé requiert toutefois des températures élevées (supérieures à 300°C) et des temps de résidence élevés, qui se traduisent par une consommation énergétique importante.

Ainsi, les techniques de traitement de gaz peuvent se classer en trois familles :
- les procédés dits récupératifs, dans lesquels le flux gazeux passe au travers de matériaux adsorbants ou absorbants. Le principe est de capter les polluants sur un support et de renouveler périodiquement ce support après encrassement.
- les procédés dits destructifs, consistant à détruire en continu les polluants présents dans le flux gazeux, par exemple par oxydations thermiques ou catalytiques, par des traitements biologiques, par photocatalyse ou par absorption avec réaction chimique.
- les procédés hybrides, à la fois récupératifs et destructifs, qui sont en deux étapes séparées. Une première étape consiste à capter les polluants sur un support, tandis qu'une seconde étape consiste à les détruire tout en régénérant le support. Durant la seconde étape, la destruction s'opère grâce à l'émission contrôlée d'espèces oxydantes comme l'ozone, l'oxygène atomique, les radicaux hydroxyles, ces espèces étant très réactives et peu sélectives au regard de la nature des espèces chimiques à détruire.

Dans les procédés récupératifs et destructifs, la seconde étape (l'étape de destruction des polluants) repose en général sur la mise en oeuvre d'une source de plasma non thermique à pression atmosphérique.

Un plasma non thermique (aussi appelé plasma froid) repose sur l'accélération de façon sélective des électrons pour que leur température atteigne 10000 à 250000K alors que la température du gaz reste sensiblement inchangée. La collision des électrons avec les molécules O2, N2 et H2O présentes dans le gaz entraine la production de molécules excitées (N2^{∗}, 02^{∗}, 03^{∗}, OH^{∗}, etc.). Ces molécules excitées émettent des photons ou de la chaleur pour perdre cette énergie. Cette énergie forme des espèces réactives et des radicaux instables (OH^{•} et O^{•}) qui contribuent aux réactions d'oxydation et ont ainsi la capacité de décomposer les polluants en des espèces moins dangereuses telles que C02, H20, HX et X2 (X étant un halogène).

En sortie des réacteurs plasma de ce type, les espèces stables les plus communément observées comprennent l'ozone et des oxydes d'azote, tels que NO, NO2, N2O, et HNO3.

Des constatations analogues peuvent être faites dans le cas d'autres types de générateurs d'ozone tels que les sources de rayonnement ultra-violet.

Il est connu en outre d'associer des générateurs d'ozone avec des catalyseurs et/ou avec une source de chaleur, afin d'obtenir une oxydation plus poussée des polluants.

Les documents WO2004/014439 et FR2972932 divulguent des systèmes présentant les caractéristiques données en introduction et mettant en oeuvre des procédés hybrides au moyen d'une source de plasma non thermique comme expliqué ci-dessus.

Les inventeurs ont cependant fait le constat que ces systèmes présentent certains inconvénients, en particulier une faible durée de vie, et un risque de libérer des oxydes d'azote dans l'air environnant.

### EXPOSÉ DE L'INVENTION

L'invention a notamment pour but d'éviter au moins en partie les inconvénients précités.

À cet effet, le système proposé par l'invention comprend en outre un second dispositif d'adsorption adapté pour adsorber les substances prédéfinies, disposé dans la portion de traitement du chemin principal, en aval du ventilateur eu égard au sens de circulation prédéterminé, et de sorte que les premier et second dispositifs d'adsorption soient disposés respectivement en amont et en aval du générateur d'ozone eu égard au sens de circulation prédéterminé.

Les inventeurs ont fait l'analyse que la faible durée de vie des systèmes de traitement de gaz de types connus était au moins en partie imputable aux phénomènes de corrosion des parties métalliques, notamment le ou les ventilateurs, et au vieillissement de certains polymères entrant dans la constitution de tels systèmes, au contact d'espèces oxydantes parmi lesquelles l'ozone et d'autres espèces susceptibles d'être produites par le générateur d'ozone.

L'agencement du générateur d'ozone entre deux dispositifs d'adsorption au sein de la portion de traitement, comme le propose l'invention dans son aspect le plus général, permet de limiter l'exposition du ventilateur aux espèces oxydantes induites par le fonctionnement du générateur d'ozone.

Dans des modes de réalisation préférés de l'invention, le système peut présenter une ou plusieurs des caractéristiques suivantes, prise(s) isolément ou suivant toutes les combinaisons techniquement possibles :
- le chemin secondaire comprend une portion active configurée pour modifier la composition du flux gazeux de recirculation, dans le mode de régénération ;
- la portion active comprend un ensemble de traitement comprenant un dispositif de catalyse apte à catalyser au moins une réaction parmi des réactions d'oxydation des substances prédéfinies, et dans lequel le ventilateur, le premier dispositif d'adsorption, le générateur d'ozone, le second dispositif d'adsorption et l'ensemble de traitement sont agencés dans cet ordre eu égard au sens de circulation prédéterminé ;
- l'ensemble de traitement comprend en outre un dispositif de traitement d'ozone adapté pour piéger ou décomposer l'ozone, et agencé en aval du dispositif de catalyse eu égard au sens de circulation prédéterminé ;
- l'ensemble de traitement comprend en outre un dispositif de piégeage adapté pour piéger au moins un produit d'au moins une réaction parmi des réactions d'oxydation des substances prédéfinies, et agencé en aval du dispositif de traitement d'ozone eu égard au sens de circulation prédéterminé ;
- la portion active comprend un dispositif de traitement d'oxydes d'azote adapté pour piéger des oxydes d'azote (NOx) ;
- le dispositif de traitement d'oxydes d'azote est agencé entre le second dispositif d'adsorption et l'ensemble de traitement ;
- le chemin secondaire est un premier chemin secondaire, la portion active est une première portion active, et la boucle de recirculation est une première boucle de recirculation, le système comprenant en outre un deuxième chemin secondaire ne comprenant pas la première portion active et présentant deux extrémités opposées raccordées au chemin principal respectivement en amont et en aval de la portion de traitement de sorte que le deuxième chemin secondaire et au moins la portion de traitement du chemin principal forment une deuxième boucle de recirculation permettant une circulation en boucle du flux gazeux de recirculation dans la portion de traitement du chemin principal, dans le mode de régénération, le deuxième chemin secondaire comprenant une deuxième portion active non inclue dans le premier chemin secondaire et comportant au moins un filtre à particules, le dispositif de commutation étant configuré pour faire se succéder des phases de recirculation dans lesquelles le flux gazeux de recirculation circule respectivement dans la première boucle de recirculation et dans la deuxième boucle de recirculation, dans le mode de régénération ;
- le système comprend en outre un troisième chemin secondaire ne comprenant pas les portions actives respectives du premier chemin secondaire et du deuxième chemin secondaire et présentant deux extrémités opposées raccordées au chemin principal respectivement en amont et en aval de la portion de traitement de sorte que le
troisième chemin secondaire et au moins la portion de traitement du chemin principal forment une troisième boucle de recirculation permettant une circulation en boucle du flux gazeux de recirculation dans la portion de traitement du chemin principal, dans le mode de régénération, le troisième chemin secondaire comprenant une troisième portion active non inclue dans le premier chemin secondaire ni dans le deuxième chemin secondaire et configurée pour modifier la composition du flux gazeux de recirculation, dans le mode de régénération, le dispositif de commutation étant configuré pour faire se succéder des phases de recirculation dans lesquelles le flux gazeux de recirculation circule respectivement dans la première boucle de recirculation, dans la troisième boucle de recirculation, et dans la deuxième boucle de recirculation, dans le mode de régénération ;
- la troisième portion active comprend un dispositif de traitement d'oxydes d'azote adapté pour piéger des oxydes d'azote (NOx) ;
- le ventilateur est logé dans la portion de traitement du chemin principal et est configuré pour propulser le flux gazeux principal au sein du chemin principal depuis l'entrée jusqu'à la sortie, dans le mode de traitement.

L'invention concerne également un procédé de traitement de gaz au moyen d'un système du type décrit ci-dessus, comprenant, en alternance :
- la mise en oeuvre du système dans le mode de traitement, dans lequel le dispositif de commutation maintient l'entrée et la sortie ouvertes, empêche la circulation du flux gazeux principal au travers du chemin secondaire, et maintient le générateur d'ozone à l'arrêt, moyennant quoi le flux gazeux principal est admis par l'entrée, puis passe dans les premier et second dispositifs d'adsorption, dans lesquels tout ou partie des substances prédéfinies éventuellement présentes dans le flux gazeux principal sont retenues par adsorption, puis le flux gazeux principal appauvri en les éventuelles substances prédéfinies est rejeté par la sortie ; et
- la mise en oeuvre du système dans le mode de régénération, dans lequel le dispositif de commutation maintient l'entrée et la sortie fermées, autorise la circulation du flux gazeux de recirculation dans le chemin secondaire, et maintient le générateur d'ozone en fonctionnement, moyennant quoi :
   a) le flux gazeux de recirculation entraîné par le ventilateur dans la boucle de recirculation se charge d'ozone produit par le générateur d'ozone et emporte l'ozone, et d'éventuelles espèces actives induites par l'ozone, dans le second dispositif d'adsorption, dans lequel d'éventuelles substances prédéfinies préalablement adsorbées par le second dispositif d'adsorption sont oxydées et désorbées au contact de l'ozone et des éventuelles espèces actives induites par l'ozone,
   b) le premier dispositif d'adsorption reçoit de l'ozone, et d'éventuelles espèces actives induites par l'ozone, se rétrodiffusant éventuellement dans le flux gazeux de recirculation, moyennant quoi d'éventuelles substances prédéfinies préalablement adsorbées par le premier dispositif d'adsorption sont oxydées et désorbées au contact de l'ozone et des éventuelles espèces actives induites par l'ozone.

### BRÈVE DESCRIPTION DES DESSINS

L'invention sera mieux comprise, et d'autres détails, avantages et caractéristiques de celle-ci apparaîtront à la lecture de la description suivante faite à titre d'exemple non limitatif et en référence aux dessins annexés dans lesquels :
- la figure 1 est un schéma fonctionnel d'un système de traitement de gaz selon un premier mode de réalisation préféré de l'invention, illustré en fonctionnement dans le mode de traitement ;
- la figure 2 est une vue semblable à la figure 1, illustrant le système en fonctionnement dans le mode de régénération ;
- les figures 3 et 4 sont des vues semblables à la figure 1, illustrant un système de traitement de gaz selon un deuxième mode de réalisation préféré de l'invention, en fonctionnement respectivement dans deux phases de son mode de régénération ;
- les figures 5 à 7 sont des vues semblables à la figure 1, illustrant un système de traitement de gaz selon un troisième mode de réalisation préféré de l'invention, en fonctionnement respectivement dans trois phases de son mode de régénération.

### EXPOSÉ DÉTAILLÉ DE MODES DE RÉALISATION PRÉFÉRÉS

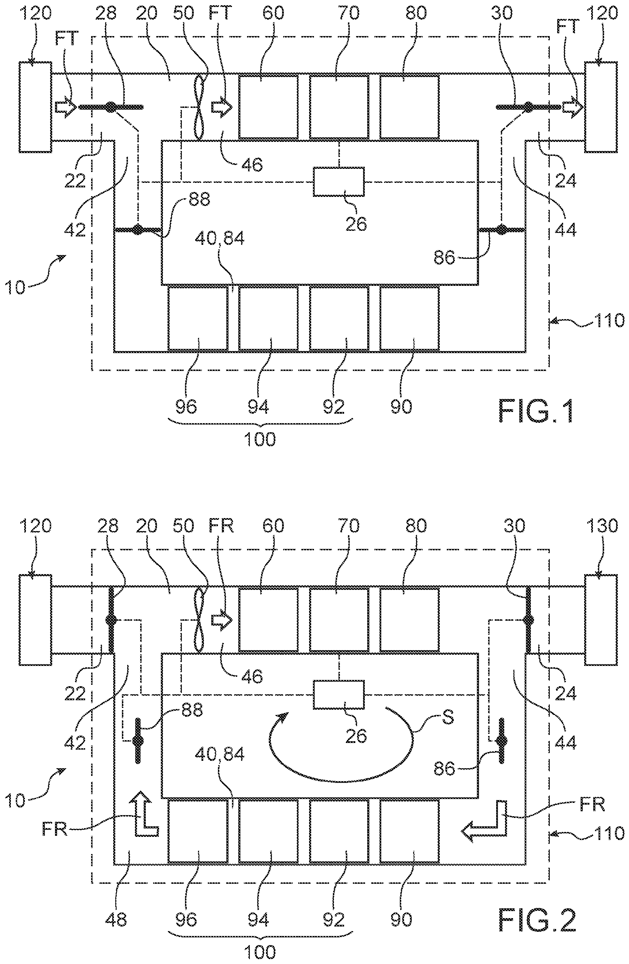

Les figures 1 et 2 illustrent schématiquement un système 10 de traitement de gaz selon un mode de réalisation préféré de l'invention, en l'occurrence un système destiné à l'épuration de l'air intérieur de bâtiments.

Le système 10 est destiné à fonctionner de manière alternée dans un mode de traitement, illustré sur la figure 1, et dans un mode de régénération, illustré sur la figure 2, comme cela apparaîtra plus clairement dans ce qui suit.

Le système 10 comprend un chemin principal 20 reliant une entrée 22 du système à une sortie 24 du système. Dans le mode de traitement, le système 10 est configuré pour permettre une circulation d'un flux gazeux FT dans le chemin principal 20 depuis l'entrée 22 jusqu'à la sortie 24, et débarrasser le flux gazeux FT d'une ou plusieurs substances prédéfinies telles que des polluants, en particulier des composés organiques volatils, au sein du chemin principal 20.

Dans ce qui précède et dans ce qui suit, un « chemin » est, de manière générale, une voie d'écoulement de gaz définie au sein d'un canal ou d'un ensemble de canaux raccordés bout-à-bout, un même canal pouvant participer à la constitution de plusieurs chemins.

Le système 10 comprend en outre un dispositif de commutation adapté pour faire passer le système du mode de traitement vers le mode de régénération, et réciproquement. Le dispositif de commutation est par exemple configuré pour opérer le changement de mode de manière automatique et régulière, par exemple de manière à maintenir le système 10 dans le mode de traitement pendant 23 heures par jour et dans le mode de régénération pendant 1 heure par jour.

Le dispositif de commutation comprend une vanne d'entrée 28 agencée dans l'entrée 22, et une vanne de sortie 30 agencée dans la sortie 24. Par « vanne », il faut ici comprendre tout dispositif apte à interrompre et rétablir sur commande la circulation d'un flux gazeux, respectivement en adoptant une configuration fermée et une configuration ouverte. Un tel dispositif est parfois dénommé « registre aéraulique ».

Le dispositif de commutation comprend en outre une unité de commande 26 pour commander les vannes d'entrée 28 et de sortie 30 de sorte que ces dernières maintiennent l'entrée 22 et la sortie 24 ouvertes dans le mode de traitement (figure 1), et maintiennent l'entrée 22 et la sortie 24 fermées (de façon étanche) dans le mode de régénération (figure 2).

Le système 10 comprend en outre un chemin secondaire 40 séparé du chemin principal 20 et présentant deux extrémités opposées 42, 44 raccordées au chemin principal 20 en deux zones distantes l'une de l'autre situées respectivement en amont et en aval d'une portion de traitement 42 définie au sein du chemin principal 20. Ainsi, le chemin secondaire 40 et la portion de traitement 46 (ou une portion plus étendue du chemin principal, incluant la portion de traitement 46) forment une boucle de recirculation 48, dans le mode de régénération, c'est-à-dire lorsque l'entrée 22 et la sortie 24 sont maintenues fermées par le dispositif de commutation (figure 2). Ce mode de fonctionnement permet ainsi la circulation en boucle d'un flux gazeux de recirculation FR dans la boucle de recirculation 48, et donc en particulier dans la portion de traitement 46.

Le dispositif de commutation comprend en outre un dispositif anti-contournement commandé par l'unité de commande 26 entre une configuration de fermeture, dans laquelle le dispositif anti-contournement obstrue (de façon étanche) le chemin secondaire 40 et empêche ainsi que le flux gazeux principal FT ne contourne la portion de traitement 46 en passant par le chemin secondaire 40 dans le mode de traitement, et une configuration d'ouverture, dans laquelle le dispositif anti-contournement autorise la circulation du flux gazeux de recirculation FR dans le chemin secondaire 40, dans le mode de régénération, comme cela apparaîtra plus clairement dans ce qui suit.

Le système 10 comprend en outre un ventilateur 50, par exemple de type axial, disposé dans la boucle de recirculation 48, et configuré pour propulser le flux gazeux de recirculation FR au sein de la boucle de recirculation 48 dans un sens de circulation S prédéterminé, dans le mode de régénération (figure 2).

Dans l'exemple illustré, le ventilateur 50 est logé dans la portion de traitement 46 du chemin principal 20 et est aussi configuré pour propulser le flux gazeux principal FT au sein du chemin principal depuis l'entrée 22 jusqu'à la sortie 24, dans le mode de traitement.

Le même ventilateur 50 permet ainsi la propulsion de l'air dans les deux modes de fonctionnement du système.

Bien entendu, le système peut néanmoins être pourvu d'un ou plusieurs ventilateurs additionnels, par exemple dans le chemin secondaire 40.

Le système 10 comprend en outre un premier dispositif d'adsorption 60 et un second dispositif d'adsorption 80, disposés dans la portion de traitement 46 du chemin principal 20 en aval du ventilateur 50 eu égard au sens de circulation S (figure 2), et adaptés pour adsorber des substances prédéfinies, par exemple des composés organiques volatils (COVs), dans le mode de traitement (figure 1).

Les dispositifs d'adsorption 60 et 80 peuvent être de tout type connu de l'art antérieur. Ces dispositifs se présentent de préférence chacun sous la forme d'une structure en nid d'abeille, par exemple en dioxyde de titane, en dioxyde de zirconium, ou en oxyde de magnésium, pourvue d'un revêtement adsorbant. Ce dernier est par exemple constitué d'un mélange d'alumine, de silice, de zéolithe, d'un mélange Al/Si, et d'éventuels autres éléments tels que des nanoparticules d'un ou plusieurs métaux choisis parmi l'or, l'argent, le cuivre, le platine, le manganèse et le palladium, et l'association de tels métaux. Le revêtement adsorbant présente de préférence une épaisseur comprise entre 10 et 200 microns, préférentiellement entre 20 et 150 microns, et une taille de pores comprise entre 3 et 20 nanomètres.

Le système 10 comprend en outre un générateur d'ozone 70 disposé dans la portion de traitement 46 du chemin principal 20, et adapté pour générer de l'ozone dans le mode de régénération. Plus précisément, le générateur d'ozone 70 est agencé de sorte que les premier et second dispositifs d'adsorption 60, 80 soient disposés respectivement en amont et en aval du générateur d'ozone 70 eu égard au sens de circulation S prédéterminé (figure 2).

Le générateur d'ozone 70 est commandé par l'unité de commande 26, qui est configurée pour maintenir le générateur d'ozone 70 à l'arrêt, dans le mode de traitement, et pour le maintenir en fonctionnement, dans le mode de régénération.

Le générateur d'ozone 70 est de préférence adapté pour générer de 0,5 à 50 grammes d'ozone par heure.

Le générateur d'ozone 70 est de préférence un réacteur à plasma non thermique, par exemple du type à décharge de surface (SD).

En variante, le générateur d'ozone 70 peut être un réacteur à plasma non thermique d'un autre type, ou encore une source de rayonnement ultra-violet (UV).

En variante encore, le générateur d'ozone 70 peut comprendre plusieurs dispositifs de génération d'ozone de types différents, par exemple un réacteur à plasma non thermique et une source de rayonnement ultra-violet (UV).

Dans des modes de réalisation préférés de l'invention, le chemin secondaire 40 comprend une portion active 84 configurée pour modifier la composition du flux gazeux de recirculation FR, dans le mode de régénération comme cela apparaîtra plus clairement dans ce qui suit.

Le dispositif anti-contournement comprend dans ce cas une première vanne d'isolation 86 agencée en amont de la portion active 84 dans le chemin secondaire 40 ou en entrée de celui-ci, et une deuxième vanne d'isolation 88 agencée en aval de la portion active 84 dans le chemin secondaire 40 ou en sortie de celui-ci. Ces vannes d'isolation 86 et 88 sont commandées par l'unité de commande 26 de sorte que les vannes d'isolation 86, 88 adoptent une configuration fermée et isolent ainsi (de façon étanche) la portion active 84 du chemin secondaire 40 du chemin principal 20 dans le mode de traitement, et de sorte que les vannes d'isolation 86, 88 adoptent une configuration ouverte et mettent ainsi la portion active 84 du chemin secondaire 40 en communication avec le chemin principal 20 dans le mode de régénération. Cette configuration du dispositif anti-contournement permet d'isoler complètement la portion active 84 du chemin principal 40, dans le mode de traitement, et d'éviter ainsi que la portion active 84 n'agisse sur le flux gazeux principal FT.

Plus précisément, la portion active 84 comprend de préférence les dispositifs suivants, agencés dans cet ordre eu égard au sens de circulation S :
- un dispositif de traitement d'oxydes d'azote 90 adapté pour réduire la concentration d'oxydes d'azote (NOx) dans le flux gazeux de recirculation FR, de tels composés pouvant notamment être générés par le générateur d'ozone ;
- un dispositif de catalyse 92 apte à catalyser au moins une réaction parmi des réactions d'oxydation des substances prédéfinies ;
- un dispositif de traitement d'ozone 94 adapté pour réduire la concentration d'ozone dans le flux gazeux de recirculation FR ; et
- un dispositif de piégeage 96 adapté pour piéger au moins un produit d'au moins une réaction parmi des réactions d'oxydation des substances prédéfinies, susceptibles de résulter de réactions d'oxydation dans l'un au moins parmi le dispositif de catalyse 92 et les premier et second dispositifs d'adsorption 60 et 80. Ce dispositif de piégeage 96 est utile en ce qui concerne les produits dont la cinétique des réactions de décomposition serait trop lente pour permettre leur décomposition au cours d'un cycle de fonctionnement en mode de régénération.

Le dispositif de traitement d'oxydes d'azote 90 est de préférence une cartouche formée d'une enveloppe poreuse contenant un matériau de piégeage d'oxydes d'azote présent sous une forme dispersée, par exemple sous forme de grains, de billes, de bâtonnets, ou de fibres, ou sous forme de nid d'abeilles.

Le matériau de piégeage d'oxydes d'azote est de préférence un matériau comprenant un ou plusieurs oxydes métalliques comme des oxydes de métaux de transition, des oxydes de métaux alcalino-terreux (par exemple, de baryum ou de magnésium), ou des oxydes de terres rares (par exemple, de cérium), ou des carbonates de formule M(CO3)x où M est choisi parmi les alcalino-terreux et les lanthanides, ou encore des zéolithes, ces derniers ayant de préférence une taille de pores comprise entre 3 et 20 nanomètres.

Ce matériau se présente de préférence sous la forme de grains de diamètre compris entre 1 et 3 millimètres.

Le dispositif de catalyse 92 est de préférence une cartouche formée d'une enveloppe poreuse contenant un matériau de catalyse présent sous une forme dispersée, par exemple sous forme de grains ou de particules ou de fibres, ou sous forme de nid d'abeilles.

Le matériau de catalyse est de préférence un catalyseur de réaction d'oxydation à basse température (par exemple entre 20 et 100 degrés Celsius), composé de particules métalliques choisies parmi l'or, l'argent, le cuivre, le manganèse, le platine et le palladium, ou d'un mélange de telles particules, dispersées sur un oxyde tel que l'alumine, la silice, le dioxyde de titane, le dioxyde de zirconium, et l'oxyde de magnésium ou un mélange de tels oxydes.

Ce matériau se présente de préférence sous la forme de grains de diamètre compris entre 1 et 3 millimètres.

Le dispositif de traitement d'ozone 94 est de préférence une cartouche formée d'une enveloppe poreuse contenant un matériau de traitement d'ozone présent sous une forme dispersée, par exemple sous forme de grains ou de particules ou de fibres, ou sous forme de nid d'abeilles.

Par « traitement d'ozone », il faut comprendre soit un piégeage de l'ozone, soit la catalyse d'une réaction de conversion de l'ozone en d'autres composés (considérés comme non nocifs ou moins nocifs).

Le matériau de traitement d'ozone est de préférence un matériau apte à décomposer l'ozone, comme un mélange de MnOx et CuOy (x et y étant des nombres entiers), éventuellement dispersé sur des oxydes comme l'alumine ou la silice, ou sur une zéolithe, de manière à présenter une surface spécifique comprise entre 10 et 1000 m2/g, préférablement entre 150 et 600 m2/g.

Ce matériau se présente de préférence sous la forme de grains de diamètre compris entre 1 et 3 millimètres.

Enfin, le dispositif de piégeage 96 est de préférence une cartouche formée d'une enveloppe poreuse contenant un matériau de piégeage présent sous une forme dispersée, par exemple sous forme de grains ou de particules ou de fibres, ou sous forme de nid d'abeilles.

Le matériau de piégeage est de préférence du charbon activé.

Dans la terminologie de l'invention, les dispositifs de catalyse 92, de traitement d'ozone 94 et de piégeage 96 constituent un ensemble de traitement 100, agencé en aval du dispositif de traitement d'oxydes d'azote 90 eu égard au sens de circulation S.

L'agencement du dispositif de traitement d'oxydes d'azote 90 en amont de l'ensemble de traitement 100 est particulièrement avantageux du fait que les oxydes d'azote 90 sont susceptibles de nuire au fonctionnement de l'ensemble de traitement 100. Ainsi, la réduction de la concentration en NOx en amont des dispositifs de catalyse 92 et de traitement d'ozone 94 permet d'éviter que des réactions impliquant les NOx n'entrent en compétition avec des réactions impliquant les substances prédéfinies, en l'occurrence des COVs, et l'ozone, au niveau de ces deux dispositifs.

De manière analogue, l'agencement du dispositif de traitement d'ozone 94 en amont du dispositif de piégeage 96 est particulièrement avantageux du fait que l'ozone peut être susceptible de dégrader le dispositif de piégeage 96, notamment lorsque ce dernier comprend du charbon activé comme dans l'exemple illustré. Ainsi, la réduction de la concentration en ozone en amont du dispositif de piégeage 96 permet d'éviter que l'ozone ne dégrade le dispositif de piégeage 96.

Par ailleurs, l'unité de commande 26 est de préférence configurée pour commander le ventilateur 50, de sorte que le ventilateur 50 tourne à une vitesse moins élevée dans le mode de régénération que dans le mode de traitement. Une vitesse plus faible du flux gazeux permet en effet de favoriser les phénomènes d'adsorption, de désorption, et, le cas échéant, de piégeage et de catalyse.

Les éléments décrits ci-dessus du système 10 forment une unité principale 110, à laquelle peuvent être facultativement adjoints un module additionnel d'entrée 120 agencé dans l'entrée 22, et comprenant par exemple un filtre à particules, et/ou un module additionnel de sortie 130 agencé dans la sortie 24, et comprenant par exemple un générateur d'ions, et/ou un ou plusieurs filtres ou dispositifs adsorbants additionnels, et/ou un autre filtre à particules.

En fonctionnement, le système 10 permet la mise en oeuvre d'un procédé de traitement de gaz, par exemple un procédé d'épuration de l'air intérieur de bâtiments, dans lequel le mode de traitement et le mode de régénération sont mis en oeuvre en alternance.

Dans le mode de traitement, le ventilateur 50 propulse le flux gazeux principal FT de l'entrée 22 jusqu'à la sortie 24, qui sont maintenues ouvertes, le générateur d'ozone 70 étant à l'arrêt, et la portion active 84 du chemin secondaire 40 étant isolée du chemin principal 20 par les vannes d'isolation 86 et 88 (figure 1), ou, plus généralement, la circulation du flux gazeux principal FT étant empêchée au travers du chemin secondaire 40 par le dispositif anti-contournement en configuration de fermeture.

Les substances prédéfinies, par exemple les COVs, éventuellement présentes dans le flux gazeux principal FT, sont adsorbées par les dispositifs d'adsorption 60 et 80, de sorte que le gaz, en l'occurrence l'air, rejeté par la sortie 24, est purifié ou tout au moins appauvri en ces substances.

Régulièrement, pour éviter la saturation des dispositifs d'adsorption 60 et 80, le système 10 passe en mode de régénération, moyennant la fermeture de l'entrée 22 et de la sortie 24 respectivement par les vannes d'entrée 28 et de sortie 30, le passage du dispositif anti-contournement en configuration d'ouverture, en l'occurrence l'ouverture des vannes d'isolation 86 et 88, permettant la mise en communication de la portion active 84 du chemin secondaire 40 avec le chemin principal 20, et la mise en fonctionnement du générateur d'ozone 70, par le dispositif de commutation 26. Le cas échéant, la source de chaleur est mise en fonctionnement.

Dans ce mode de fonctionnement, le ventilateur 50 force la circulation du flux gazeux de recirculation FR dans la boucle de recirculation 48. À cet effet, la puissance de fonctionnement du ventilateur 50 est de préférence réduite par rapport à la puissance de celui-ci dans le mode de traitement.

Une partie, en principe majoritaire, de l'ozone généré par le générateur d'ozone 70, est transportée vers le second dispositif d'adsorption 80 par le flux gazeux de recirculation FR, tandis qu'une autre partie, en principe minoritaire, de l'ozone généré par le générateur d'ozone 70, se déplace vers le premier dispositif d'adsorption 60 par rétrodiffusion.

Des réactions d'oxydation des substances prédéfinies, préalablement adsorbées par les dispositifs d'adsorption 60 et 80, se produisent au sein de ces deux dispositifs, du fait de la présence, au sein de ces dispositifs, de l'ozone et/ou d'éventuelles autres espèces actives induites par le fonctionnement du générateur d'ozone 70, comme expliqué ci-dessus. Il en résulte une régénération progressive des dispositifs d'adsorption 60 et 80, moyennant la libération des sites d'adsorption, par désorption de substances prédéfinies et/ou de composés résultant de l'oxydation de ces substances prédéfinies sur les sites d'adsorption.

L'agencement du premier dispositif d'adsorption 60 entre le ventilateur 50 et le générateur d'ozone 70 permet en particulier de limiter l'exposition du ventilateur 50 à l'ozone, et aux éventuelles autres espèces oxydantes, se rétrodiffusant vers l'amont, eu égard au sens de circulation S, à partir du générateur d'ozone 70.

Le flux gazeux de recirculation FR passe ensuite dans le dispositif de traitement d'oxydes d'azote 90, qui permet de réduire la concentration en oxydes d'azote dans ce flux gazeux de recirculation FR.

Le flux gazeux de recirculation FR, appauvri en oxydes d'azote, ou dépourvu de ces derniers, passe ensuite dans l'ensemble de traitement 100.

Le flux gazeux de recirculation FR rencontre en premier lieu le dispositif de catalyse 92, qui permet de maximiser l'oxydation des substances prédéfinies par l'ozone et/ou par les éventuelles autres espèces oxydantes induites par le fonctionnement du générateur d'ozone 70.

Puis le flux gazeux de recirculation FR rencontre le dispositif de traitement d'ozone 94, qui permet de réduire drastiquement la concentration en ozone dans le flux gazeux de recirculation, en décomposant (ou, en variante, en piégeant) l'ozone.

Enfin, le flux gazeux de recirculation FR rencontre le dispositif de piégeage 96 qui permet de piéger d'éventuels produits d'oxydation préalablement générés dans l'un au moins parmi le dispositif de catalyse 92 et les premier et second dispositifs d'adsorption 60 et 80. Les produits d'oxydation considérés sont donc de manière générale issus de réactions d'oxydation des substances prédéfinies.

Après avoir parcouru l'intégralité de la boucle de recirculation 48, le flux gazeux de recirculation FR rencontre de nouveau le ventilateur 50 et poursuit sa circulation en boucle de la manière décrite ci-dessus.

L'agencement du dispositif de traitement d'ozone 94 dans le chemin secondaire 40 permet de limiter l'exposition du ventilateur 50 à la part de l'ozone emportée par le flux gazeux de recirculation FR à partir du générateur d'ozone 70 et subsistant dans ce flux gazeux de recirculation en sortie du second dispositif d'adsorption 80.

Les inventeurs ont fait l'analyse que la faible durée de vie des systèmes de traitement de gaz de types connus était au moins en partie imputable aux phénomènes de corrosion des parties métalliques et d'oxydation de parties réalisées en polymère, notamment le ou les ventilateurs, par des espèces oxydantes dont en premier lieu l'ozone, mais également les oxydes d'azote.

L'agencement du premier dispositif d'adsorption 60 entre le ventilateur 50 et le générateur d'ozone 70 au sein du chemin principal 20, en amont du générateur d'ozone eu égard au sens de circulation S, d'une part, et l'agencement du dispositif de traitement d'ozone 94 dans le chemin secondaire 40, en aval du générateur d'ozone eu égard au sens de circulation S, d'autre part, permettent de limiter l'exposition du ventilateur 50 à l'ozone et aux éventuelles autres substances oxydantes, et donc d'accroître la durée de vie de celui-ci.

Ces caractéristiques permettent, en outre, de limiter la concentration en ozone dans une majeure partie de la boucle de recirculation 48, et donc de limiter l'exposition à l'ozone d'éventuelles autres parties du système réalisées en métal ou en polymère.

Par ailleurs, la présence du dispositif de traitement d'oxydes d'azote 90 au sein de la boucle de recirculation 48, en aval du générateur d'ozone 70, permet de limiter l'exposition du ventilateur 50 (et d'éventuelles autres parties métalliques ou en polymère) aux oxydes d'azote, et donc là encore d'accroître la durée de vie du ventilateur 50 (et des éventuelles autres parties métalliques).

Le dispositif de traitement d'oxydes d'azote 90 permet en outre de minimiser le risque que le gaz rejeté par la sortie 24, dans le mode de traitement, ne contienne des oxydes d'azote générés au cours d'une phase de fonctionnement antérieure en mode de régénération.

Des avantages analogues découlent de la présence, dans la portion active 84 du chemin secondaire 40, du dispositif de piégeage 96, dès lors que certains produits d'oxydation, susceptibles de demeurer présents en sortie du dispositif de traitement d'ozone 94, peuvent présenter un caractère corrosif et/ou nocif s'ils venaient à être rejetés par le système.

Le système 10 décrit ci-dessus implique un traitement, dans une même boucle de recirculation, de l'ensemble des substances prédéfinies et des éventuelles autres espèces à traiter induites par le fonctionnement du générateur d'ozone 70.

Dans certains cas, il peut pourtant être avantageux de traiter successivement différentes substances ou espèces au moyen de plusieurs boucles de recirculation, chaque boucle de recirculation pouvant être configurée pour un traitement générique, c'est-à-dire visant toutes les substances prédéfinies et les éventuelles autres espèces à traiter, ou pour un traitement spécifique à une ou plusieurs des substances prédéfinies et des éventuelles autres espèces à traiter. Des boucles de recirculation dédiées à des traitements spécifiques présentent notamment l'avantage d'une perte de charge réduite par rapport à une boucle de recirculation comprenant l'ensemble des traitements.

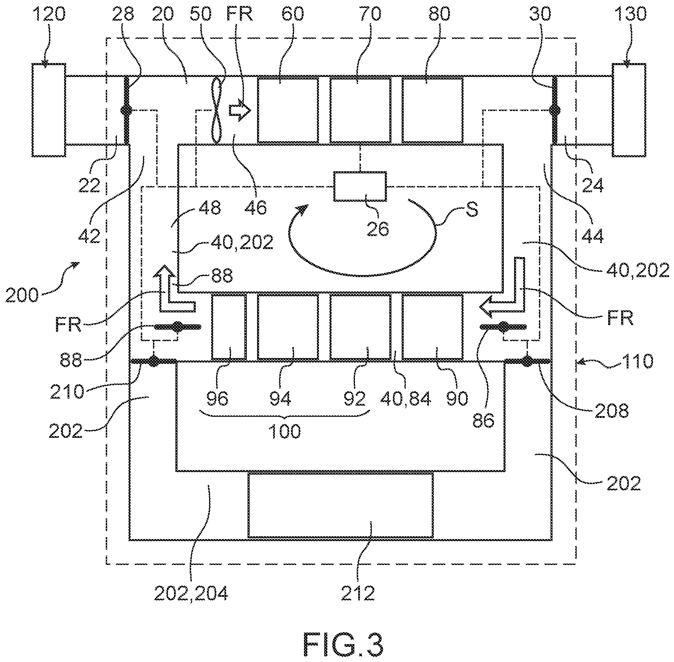

À cet effet, les figures 3 à 7 illustrent des systèmes de traitement de gaz selon d'autres modes de réalisation préférés de l'invention, qui se distinguent du système 10 décrit ci-dessus du fait que ces systèmes comprennent plusieurs chemins secondaires.

Dans ces systèmes, par souci de clarté, le chemin secondaire 40, la portion active 84, et la boucle de recirculation 48, sont respectivement dénommés premier chemin secondaire, première portion active, et première boucle de recirculation.

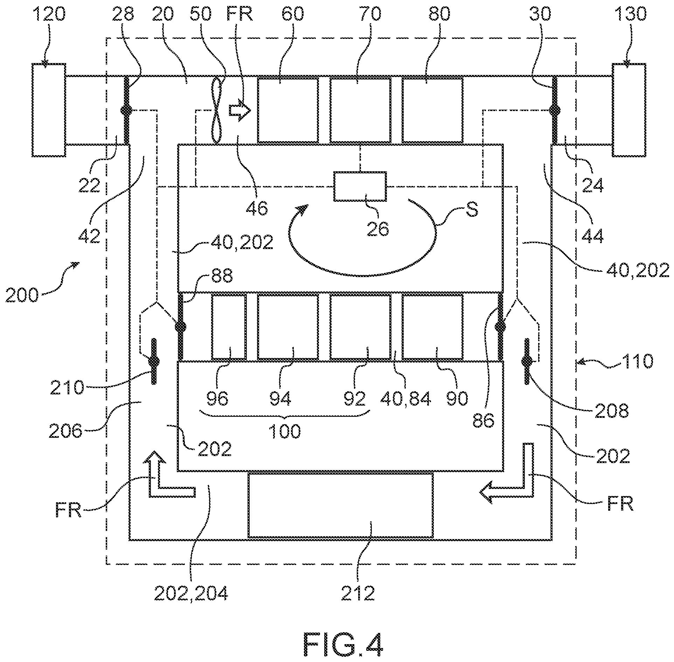

Le système de traitement de gaz 200 illustré sur les figures 3 et 4 comporte un deuxième chemin secondaire 202 ne comprenant pas la première portion active 84 et présentant deux extrémités opposées raccordées au chemin principal 20 respectivement en amont et en aval de la portion de traitement 46. Dans l'exemple illustré, les deux extrémités du deuxième chemin secondaire 202 se confondent respectivement avec les deux extrémités 42, 44 du premier chemin secondaire 40.

Entre ses deux extrémités opposées, le deuxième chemin secondaire 202 comporte une deuxième portion active 204 non inclue dans le premier chemin secondaire 40, et configurée pour modifier la composition du flux gazeux de recirculation FR, dans le mode de régénération.

Le deuxième chemin secondaire 202 et au moins la portion de traitement 46 du chemin principal 20 forment une deuxième boucle de recirculation 206 (figure 4) permettant une circulation en boucle du flux gazeux de recirculation FR dans la portion de traitement 46 du chemin principal 20, dans le mode de régénération.

Le dispositif de commutation 26 est configuré pour faire se succéder une première phase de recirculation et une deuxième phase de recirculation, dans lesquelles le flux gazeux de recirculation FR circule respectivement dans la première boucle de recirculation 48 (figure 3) et dans la deuxième boucle de recirculation 206 (figure 4), dans le mode de régénération.

Plus précisément, le dispositif anti-contournement est tel que dans sa configuration de fermeture, le dispositif anti-contournement obstrue en outre (de façon étanche) le deuxième chemin secondaire 202. Le dispositif anti-contournement empêche ainsi que le flux gazeux principal FT ne contourne la portion de traitement 46 en passant par le deuxième chemin secondaire 202, dans le mode de traitement.

Dans sa configuration d'ouverture, le dispositif anti-contournement autorise alternativement une circulation du flux gazeux de recirculation FR au travers du premier chemin secondaire 40 et du deuxième chemin secondaire 202.

À cet effet, les première et deuxième vannes d'isolation 86 et 88 sont agencées au sein du premier chemin secondaire 40, hors du deuxième chemin secondaire 202 (ou en limite de ce dernier). De plus, le dispositif anti-contournement comprend en outre une troisième vanne d'isolation 208 agencée en amont de la deuxième portion active 204 dans le deuxième chemin secondaire 202, hors du premier chemin secondaire 40 (ou en limite de ce dernier), et une quatrième vanne d'isolation 210 agencée en aval de la deuxième portion active 204 dans le deuxième chemin secondaire 202, également hors du premier chemin secondaire 40 (ou en limite de ce dernier).

Les troisième et quatrième vannes d'isolation 208 et 210 sont commandées par l'unité de commande 26 de sorte que :
- les troisième et quatrième vannes d'isolation 208 et 210 isolent (de façon étanche) la deuxième portion active 204 du deuxième chemin secondaire 202 du chemin principal 20, dans le mode de traitement, et isolent (de façon étanche) la deuxième portion active 204 du deuxième chemin secondaire 202 de la première boucle de recirculation 48, dans la première phase de recirculation (figure 3), et de sorte que
- les troisième et quatrième vannes d'isolation 208 et 210 mettent la deuxième portion active 204 du deuxième chemin secondaire 202 en communication avec le chemin principal 20 dans la deuxième phase de recirculation (figure 4).

Cette configuration du dispositif anti-contournement permet d'éviter que la deuxième portion active 204 n'agisse sur le flux gazeux principal FT dans le mode de traitement et que la deuxième portion active 204 n'agisse sur le flux gazeux de recirculation FR dans la première phase de recirculation.

De plus, les première et deuxième vannes d'isolation 86 et 88 sont commandées par l'unité de commande 26 de sorte que les première et deuxième vannes d'isolation 86 et 88 isolent (de façon étanche) la première portion active 84 du premier chemin secondaire 40 de la deuxième boucle de recirculation 206, dans la deuxième phase de recirculation (figure 4).

Le dispositif anti-contournement permet ainsi d'éviter que la première portion active 84 n'agisse sur le flux gazeux de recirculation FR dans la deuxième phase de recirculation.

Dans le mode de réalisation illustré, la deuxième portion active 204 comporte un filtre à particules 212.

En effet, pendant le fonctionnement du système en mode de régénération, il peut se produire la formation d'aérosols organiques du fait de la dégradation chimique de certains composés organiques. Par exemple, dans le cas du traitement de composés organiques volatils, des réactions par les oxydes d'azote (NOx) ou par l'ozone (O3) peuvent aboutir à la formation d'espèces semi-volatiles, c'est-à-dire dont les tensions de vapeur sont faibles. Ces produits peuvent se déposer à la surface de particules pré-existantes (selon un phénomène de condensation hétérogène) ou former de nouvelles particules (selon un phénomène de nucléation homogène ou de conversion gaz-particule).

Le filtre à particules 212 permet de capter de telles particules et d'éviter ainsi la contamination du milieu extérieur par ces particules.

Un tel filtre peut être formé d'un matériau fibreux ou électrostatique.

Le système 200 fonctionne donc de manière analogue au fonctionnement du système 10 des figures 1 et 2, sauf en ce qui concerne le mode de régénération. Dans le cas du système 200, ce mode de régénération comprend successivement :
- la première phase de recirculation, dans laquelle les première et deuxième vannes d'isolation 86 et 88 sont ouvertes tandis que les troisième et quatrième vannes d'isolation 208 et 210 sont fermées, moyennant quoi le flux gazeux de recirculation FR circule dans la première boucle de recirculation 48 (figure 3), et voit sa composition modifiée de la manière décrite ci-dessus en référence au système 10 ; et
- la deuxième phase de recirculation, dans laquelle les première et deuxième vannes d'isolation 86 et 88 sont fermées tandis que les troisième et quatrième vannes d'isolation 208 et 210 sont ouvertes, moyennant quoi le flux gazeux de recirculation FR circule dans la deuxième boucle de recirculation 206 (figure 4), dans laquelle le filtre à particules 212 permet de capter les éventuelles particules précitées.

Ces deux phases de recirculation peuvent éventuellement être mises en oeuvre plusieurs fois en alternance, avant un retour au mode de traitement.

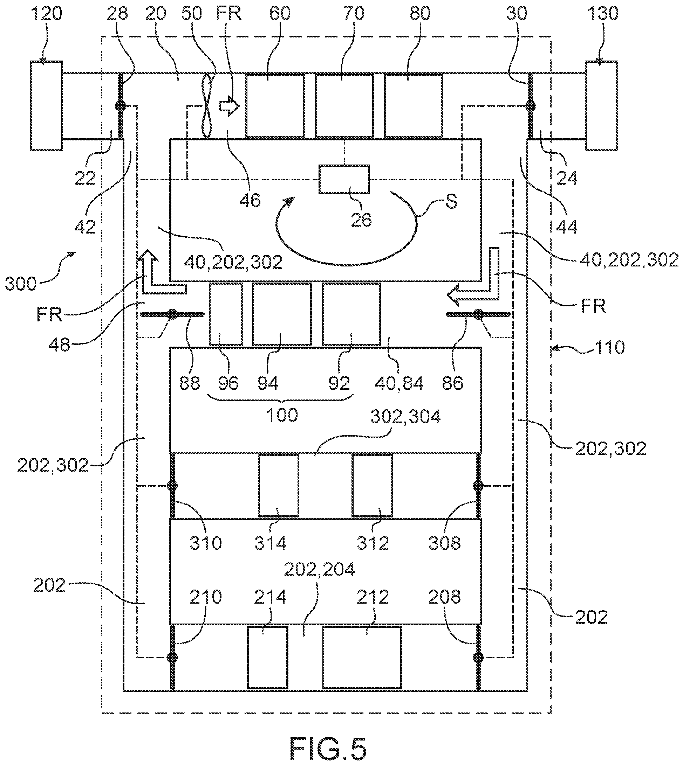

Le système de traitement de gaz 300 illustré sur les figures 5 à 7 est semblable au système 200 mais se distingue de ce dernier du fait que le système 300 comporte en outre un troisième chemin secondaire 302 ne comprenant pas les portions actives respectives 84, 204 du premier chemin secondaire 40 et du deuxième chemin secondaire 202, et présentant deux extrémités opposées raccordées au chemin principal 20 respectivement en amont et en aval de la portion de traitement 46. Dans l'exemple illustré, les deux extrémités du troisième chemin secondaire 302 se confondent respectivement avec les deux extrémités 42, 44 du premier chemin secondaire 40.

Entre ses deux extrémités opposées, le troisième chemin secondaire 302 comporte une troisième portion active 304 non inclue dans le premier chemin secondaire 40 ni dans le deuxième chemin secondaire 202, et configurée pour modifier la composition du flux gazeux de recirculation FR, dans le mode de régénération.

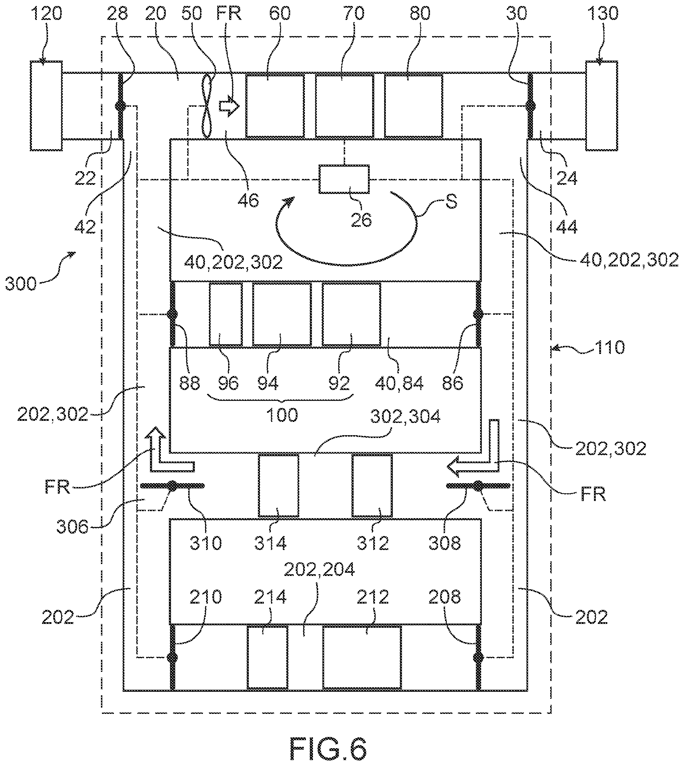

Le troisième chemin secondaire 302 et au moins la portion de traitement 46 du chemin principal 20 forment une troisième boucle de recirculation 306 (figure 6) permettant une circulation en boucle du flux gazeux de recirculation FR dans la portion de traitement 46 du chemin principal 20, dans le mode de régénération.

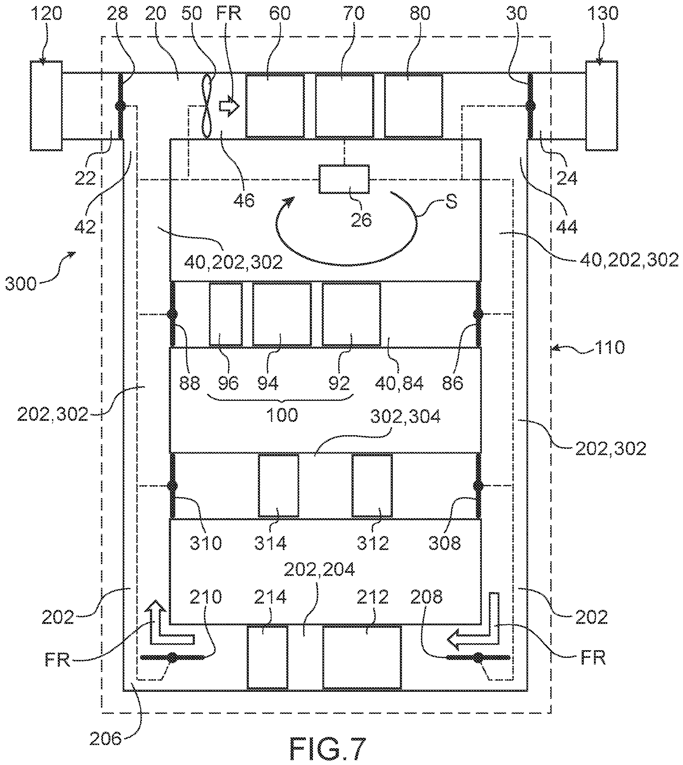

Le dispositif de commutation 26 est configuré pour faire se succéder une première phase de recirculation, une deuxième phase de recirculation, et une troisième phase de recirculation, dans lesquelles le flux gazeux de recirculation FR circule respectivement dans la première boucle de recirculation 48 (figure 5), dans la troisième boucle de recirculation 306 (figure 6), et dans la deuxième boucle de recirculation 206 (figure 7), dans le mode de régénération.

Plus précisément, le dispositif anti-contournement est tel que dans sa configuration de fermeture, le dispositif anti-contournement obstrue en outre (de façon étanche) le troisième chemin secondaire 302. Le dispositif anti-contournement empêche ainsi que le flux gazeux principal FT ne contourne la portion de traitement 46 en passant par le troisième chemin secondaire 302, dans le mode de traitement.

Dans sa configuration d'ouverture, le dispositif anti-contournement autorise alternativement une circulation du flux gazeux de recirculation FR au travers du premier chemin secondaire 40, du troisième chemin secondaire 302, et du deuxième chemin secondaire 202.

À cet effet, les première et deuxième vannes d'isolation 86 et 88 sont agencées au sein du premier chemin secondaire 40, hors du deuxième chemin secondaire 202 (ou en limite de ce dernier) et hors du troisième chemin secondaire 302 (ou en limite de ce dernier). De plus, les troisième et quatrième vannes d'isolation 208 et 210 sont agencées au sein du deuxième chemin secondaire 202, hors du premier chemin secondaire 40 (ou en limite de ce dernier), et hors du troisième chemin secondaire 302 (ou en limite de ce dernier). Le dispositif anti-contournement comprend en outre une cinquième vanne d'isolation 308 agencée en amont de la troisième portion active 304 dans le troisième chemin secondaire 302, hors du premier chemin secondaire 40 (ou en limite de ce dernier) et hors du deuxième chemin secondaire 202 (ou en limite de ce dernier), et une sixième vanne d'isolation 310 agencée en aval de la troisième portion active 304 dans le troisième chemin secondaire 302, également hors du premier chemin secondaire 40 (ou en limite de ce dernier) et hors du deuxième chemin secondaire 202 (ou en limite de ce dernier).

Les cinquième et sixième vannes d'isolation 308 et 310 sont commandées par l'unité de commande 26 de sorte que :
- les cinquième et sixième vannes d'isolation 308 et 310 isolent (de façon étanche) la troisième portion active 304 du troisième chemin secondaire 302 du chemin principal 20 dans le mode de traitement, isolent (de façon étanche) la troisième portion active 304 du troisième chemin secondaire 302 de la première boucle de recirculation 48 dans la première phase de recirculation (figure 5), et isolent (de façon étanche) la troisième portion active 304 du troisième chemin secondaire 302 de la deuxième boucle de recirculation 206 dans la troisième phase de recirculation (figure 7), et de sorte que
- les cinquième et sixième vannes d'isolation 308 et 310 mettent la troisième portion active 304 du troisième chemin secondaire 302 en communication avec le chemin principal 20 dans la deuxième phase de recirculation (figure 6).

Cette configuration du dispositif anti-contournement permet d'éviter que la troisième portion active 304 n'agisse sur le flux gazeux principal FT dans le mode de traitement et que la troisième portion active 304 n'agisse sur le flux gazeux de recirculation FR dans les première et troisième phases de recirculation.

De plus, les première et deuxième vannes d'isolation 86 et 88 sont commandées par l'unité de commande 26 de sorte que les première et deuxième vannes d'isolation 86 et 88 isolent (de façon étanche) la première portion active 84 du premier chemin secondaire 40 de la troisième boucle de recirculation 306, dans la deuxième phase de recirculation (figure 6), et isolent la première portion active 84 du premier chemin secondaire 40 de la deuxième boucle de recirculation 206, dans la troisième phase de recirculation (figure 7).

Le dispositif anti-contournement permet ainsi d'éviter que la première portion active 84 n'agisse sur le flux gazeux de recirculation FR dans les deuxième et troisième phases de recirculation.

Enfin, les troisième et quatrième vannes d'isolation 208 et 210 sont commandées par l'unité de commande 26 de sorte que :
- les troisième et quatrième vannes d'isolation 208 et 210 isolent (de façon étanche) la deuxième portion active 204 du deuxième chemin secondaire 202 de la première boucle de recirculation 48, dans la première phase de recirculation (figure 5), et isolent (de façon étanche) la deuxième portion active 204 du deuxième chemin secondaire 202 de la troisième boucle de recirculation 306, dans la deuxième phase de recirculation (figure 6), et de sorte que
- les troisième et quatrième vannes d'isolation 208 et 210 mettent la deuxième portion active 204 du deuxième chemin secondaire 202 en communication avec le chemin principal 20 dans la troisième phase de recirculation (figure 7).

Cette configuration du dispositif anti-contournement permet d'éviter que la deuxième portion active 204 n'agisse sur le flux gazeux de recirculation FR dans la première phase de recirculation et dans la deuxième phase de recirculation.

Dans le mode de réalisation illustré, la deuxième portion active 204 comporte en outre un dispositif de piégeage 214 adapté pour piéger au moins un produit d'au moins une réaction parmi des réactions d'oxydation des substances prédéfinies, et agencé en aval du filtre à particules 212 eu égard au sens de circulation S prédéterminé.

De plus, la troisième portion active 304 comporte un dispositif de traitement d'oxydes d'azote 312 semblable au dispositif de traitement d'oxydes d'azote 90 décrit ci-dessus, auquel cas la première portion active 84 est dépourvue de ce dispositif 90.

Dans l'exemple illustré, la troisième portion active 304 comporte en outre un dispositif de piégeage 314 adapté pour piéger au moins un produit d'au moins une réaction parmi des réactions d'oxydation des substances prédéfinies, et agencé en aval du dispositif de traitement d'oxydes d'azote 312 eu égard au sens de circulation S prédéterminé.

Le système 300 fonctionne donc de manière analogue au fonctionnement du système 200 des figures 3 et 4, sauf en ce qui concerne le mode de régénération. Dans le cas du système 300, ce mode de régénération comprend successivement :
- la première phase de recirculation, dans laquelle les première et deuxième vannes d'isolation 86 et 88 sont ouvertes, tandis que les troisième, quatrième, cinquième et sixième vannes d'isolation 208, 210, 308, 310 sont fermées, moyennant quoi le flux gazeux de recirculation FR circule dans la première boucle de recirculation 48 (figure 5), et voit sa composition modifiée de la manière décrite ci-dessus en référence au système 10 sauf en ce qui concerne les oxydes d'azote (NOx) ;
- la deuxième phase de recirculation, dans laquelle les cinquième et sixième vannes d'isolation 308 et 310 sont ouvertes, tandis que les première, deuxième, troisième et quatrième vannes d'isolation 86, 88, 208 et 210 sont fermées, moyennant quoi le flux gazeux de recirculation FR circule dans la troisième boucle de recirculation 306 (figure 6), dans laquelle le dispositif de traitement d'oxydes d'azote 312 permet de réduire la concentration en oxydes d'azote dans le flux gazeux de recirculation FR ;
- la troisième phase de recirculation, dans laquelle les troisième et quatrième vannes d'isolation 208 et 210 sont ouvertes, tandis que les première, deuxième, cinquième et sixième vannes d'isolation 86, 88, 308 et 310 sont fermées, moyennant quoi le flux gazeux de recirculation FR circule dans la deuxième boucle de recirculation 206 (figure 7), dans laquelle le filtre à particules 212 permet de capter les éventuelles particules précitées.

Dans l'exemple illustré, les dispositifs de piégeage 214 et 314 permettent de capter les éventuels produits de réactions d'oxydation subsistant dans le flux gazeux de recirculation FR, dans les deuxième et troisième phases de recirculation.

Les trois phases de recirculation peuvent éventuellement être mises en oeuvre plusieurs fois en alternance, avant un retour au mode de traitement.

En variante, les différents chemins secondaires du système peuvent ne pas comporter de portions communes, et en particulier être raccordés au chemin principal 20 indépendamment les uns des autres.

## Revendications

1. Système de traitement de gaz (10, 200, 300), apte à fonctionner de manière alternée dans un mode de traitement et dans un mode de régénération, comprenant :
- une entrée (22) et une sortie (24), et un chemin principal (20) reliant l'entrée à la sortie et comprenant une portion de traitement (46), de manière à permettre la circulation d'un flux gazeux principal (FT) depuis l'entrée jusqu'à la sortie en passant par la portion de traitement, dans le mode de traitement ;
- au moins un chemin secondaire (40) présentant deux extrémités opposées (42, 44) raccordées au chemin principal (20) respectivement en amont et en aval de la portion de traitement (46), de sorte que le chemin secondaire (40) et au moins la portion de traitement (46) du chemin principal forment une boucle de recirculation (48) permettant une circulation en boucle d'un flux gazeux de recirculation (FR) dans la portion de traitement (46) du chemin principal, dans le mode de régénération ;
- un ventilateur (50) disposé dans la boucle de recirculation (48) et configuré au moins pour propulser le flux gazeux de recirculation (FR) au sein de la boucle de recirculation dans un sens de circulation (S) prédéterminé, dans le mode de régénération ;
- un premier dispositif d'adsorption (60) disposé dans la portion de traitement (46) du chemin principal, en aval du ventilateur (50) eu égard au sens de circulation (S) prédéterminé, et adapté pour adsorber des substances prédéfinies éventuellement présentes dans le flux gazeux principal (FT), dans le mode de traitement ;
- un générateur d'ozone (70) disposé dans la portion de traitement (46) du chemin principal, en aval du ventilateur (50) eu égard au sens de circulation (S) prédéterminé, et adapté pour générer de l'ozone dans le mode de régénération ; et
- un dispositif de commutation configuré pour maintenir l'entrée (22) et la sortie (24) ouvertes, empêcher la circulation du flux gazeux principal (FT) au travers du chemin secondaire (40) ou de chaque chemin secondaire (40, 202, 302), et maintenir le générateur d'ozone (70) à l'arrêt, dans le mode de traitement, et pour maintenir l'entrée (22) et la sortie (24) fermées, autoriser la circulation du flux gazeux de recirculation (FR) dans le chemin secondaire (40) ou dans chaque chemin secondaire (40, 202, 302), et maintenir le générateur d'ozone (70) en fonctionnement, dans le mode de régénération ; **caractérisé en ce qu'**il comprend en outre un second dispositif d'adsorption (80) adapté pour adsorber les substances prédéfinies, disposé dans la portion de traitement (46) du chemin principal en aval du ventilateur (50) eu égard au sens de circulation prédéterminé et de sorte que les premier et second dispositifs d'adsorption (60, 80) soient disposés respectivement en amont et en aval du générateur d'ozone (70) eu égard au sens de circulation (S) prédéterminé.

2. Système selon la revendication 1, dans lequel le chemin secondaire (40) comprend une portion active (84) configurée pour modifier la composition du flux gazeux de recirculation (FR), dans le mode de régénération.

3. Système selon la revendication 2, dans lequel la portion active comprend un ensemble de traitement (100) comprenant un dispositif de catalyse (92) apte à catalyser au moins une réaction parmi des réactions d'oxydation des substances prédéfinies, et dans lequel le ventilateur (50), le premier dispositif d'adsorption (60), le générateur d'ozone (70), le second dispositif d'adsorption (80) et l'ensemble de traitement (100) sont agencés dans cet ordre eu égard au sens de circulation (S) prédéterminé.

4. Système selon la revendication 3, dans lequel l'ensemble de traitement (100) comprend en outre un dispositif de traitement d'ozone (94) adapté pour piéger ou décomposer l'ozone, et agencé en aval du dispositif de catalyse (92) eu égard au sens de circulation (S) prédéterminé.

5. Système selon la revendication 4, dans lequel l'ensemble de traitement (100) comprend en outre un dispositif de piégeage (96) adapté pour piéger au moins un produit d'au moins une réaction parmi des réactions d'oxydation des substances prédéfinies, et agencé en aval du dispositif de traitement d'ozone (94) eu égard au sens de circulation (S) prédéterminé.

6. Système selon l'une quelconque des revendications 2 à 5, dans lequel la portion active (84) comprend un dispositif de traitement d'oxydes d'azote (90) adapté pour piéger des oxydes d'azote (NOx).

7. Système selon la combinaison des revendications 3 et 6, dans lequel le dispositif de traitement d'oxydes d'azote (90) est agencé entre le second dispositif d'adsorption (80) et l'ensemble de traitement (100).

8. Système selon l'une quelconque des revendications 2 à 5, dans lequel le chemin secondaire est un premier chemin secondaire (40), la portion active est une première portion active (84), et la boucle de recirculation est une première boucle de recirculation (48), le système comprenant en outre un deuxième chemin secondaire (202) ne comprenant pas la première portion active (84) et présentant deux extrémités opposées raccordées au chemin principal (40) respectivement en amont et en aval de la portion de traitement (46) de sorte que le deuxième chemin secondaire (202) et au moins la portion de traitement (46) du chemin principal forment une deuxième boucle de recirculation (206) permettant une circulation en boucle du flux gazeux de recirculation (FR) dans la portion de traitement (46) du chemin principal, dans le mode de régénération, le deuxième chemin secondaire (202) comprenant une deuxième portion active (204) non inclue dans le premier chemin secondaire (40) et comportant au moins un filtre à particules (212), le dispositif de commutation étant configuré pour faire se succéder des phases de recirculation dans lesquelles le flux gazeux de recirculation (FR) circule respectivement dans la première boucle de recirculation (48) et dans la deuxième boucle de recirculation (206), dans le mode de régénération.

9. Système selon la revendication 8, comprenant en outre un troisième chemin secondaire (302) ne comprenant pas les portions actives respectives (84, 204) du premier chemin secondaire (40) et du deuxième chemin secondaire (202) et présentant deux extrémités opposées raccordées au chemin principal (40) respectivement en amont et en aval de la portion de traitement (46) de sorte que le troisième chemin secondaire (302) et au moins la portion de traitement (46) du chemin principal forment une troisième boucle de recirculation (306) permettant une circulation en boucle du flux gazeux de recirculation (FR) dans la portion de traitement (46) du chemin principal, dans le mode de régénération, le troisième chemin secondaire (302) comprenant une troisième portion active (304) non inclue dans le premier chemin secondaire (40) ni dans le deuxième chemin secondaire (202) et configurée pour modifier la composition du flux gazeux de recirculation (FR), dans le mode de régénération, le dispositif de commutation étant configuré pour faire se succéder des phases de recirculation dans lesquelles le flux gazeux de recirculation (FR) circule respectivement dans la première boucle de recirculation (48), dans la troisième boucle de recirculation (306), et dans la deuxième boucle de recirculation (206), dans le mode de régénération.

10. Système selon la revendication 9, dans lequel la troisième portion active (304) comprend un dispositif de traitement d'oxydes d'azote (312) adapté pour piéger des oxydes d'azote (NOx).

11. Système selon l'une quelconque des revendications 1 à 10, dans lequel le ventilateur (50) est logé dans la portion de traitement (46) du chemin principal et est configuré pour propulser le flux gazeux principal (FT) au sein du chemin principal depuis l'entrée (22) jusqu'à la sortie (24), dans le mode de traitement.

12. Procédé de traitement de gaz au moyen d'un système (10, 200, 300) selon l'une quelconque des revendications 1 à 11, comprenant, en alternance :
- la mise en oeuvre du système dans le mode de traitement, dans lequel le dispositif de commutation maintient l'entrée (22) et la sortie (24) ouvertes, empêche la circulation du flux gazeux principal (FT) au travers du chemin secondaire (40) ou de chaque chemin secondaire (40, 202, 302), et maintient le générateur d'ozone (70) à l'arrêt, moyennant quoi le flux gazeux principal (FT) est admis par l'entrée (22), puis passe dans les premier et second dispositifs d'adsorption (60, 80), dans lesquels tout ou partie des substances prédéfinies éventuellement présentes dans le flux gazeux principal sont retenues par adsorption, puis le flux gazeux principal appauvri en les éventuelles substances prédéfinies est rejeté par la sortie (24) ; et
- la mise en oeuvre du système dans le mode de régénération, dans lequel le dispositif de commutation maintient l'entrée (22) et la sortie (24) fermées, autorise la circulation du flux gazeux de recirculation (FR) dans le chemin secondaire (40) ou dans chaque chemin secondaire (40, 202, 302), et maintient le générateur d'ozone (70) en fonctionnement, moyennant quoi :
a) le flux gazeux de recirculation (FR) entraîné par le ventilateur (50) dans la boucle de recirculation (48) se charge d'ozone produit par le générateur d'ozone (70) et emporte l'ozone, et d'éventuelles espèces actives induites par l'ozone, dans le second dispositif d'adsorption (80), dans lequel d'éventuelles substances prédéfinies préalablement adsorbées par le second dispositif d'adsorption sont oxydées et désorbées au contact de l'ozone et des éventuelles espèces actives induites par l'ozone,
b) le premier dispositif d'adsorption (60) reçoit de l'ozone, et d'éventuelles espèces actives induites par l'ozone, se rétrodiffusant éventuellement dans le flux gazeux de recirculation (FR), moyennant quoi d'éventuelles substances prédéfinies préalablement adsorbées par le premier dispositif d'adsorption (60) sont oxydées et désorbées au contact de l'ozone et des éventuelles espèces actives induites par l'ozone.

## Patentansprüche

1. Gasbehandlungssystem (10, 200, 300), das ausgelegt ist, abwechselnd in einem Behandlungsmodus und in einem Regenerationsmodus zu arbeiten, Folgendes umfassend:
- einen Eingang (22) und einen Ausgang (24) und einen Hauptpfad (20), der den Eingang mit dem Ausgang verbindet und einen Behandlungsabschnitt (46) umfasst, um die Zirkulation eines Hauptgasstroms (FT) von dem Eingang über den Behandlungsabschnitt bis zum Ausgang im Behandlungsmodus zu gestatten;
- mindestens einen sekundären Pfad (40), der zwei gegenüberliegende Enden (42, 44) aufweist, die jeweils stromaufwärts und stromabwärts des Behandlungsabschnitts (46) mit dem Hauptpfad (20) derart verbunden sind, dass der sekundäre Pfad (40) und mindestens der Behandlungsabschnitt (46) des Hauptpfads eine Rezirkulationsschleife (48) bilden, die eine Schleifenzirkulation eines Rezirkulationsgasstroms (FR) in dem Behandlungsabschnitt (46) des Hauptpfads im Regenerationsmodus gestattet;
- ein Gebläse (50), das in der Rezirkulationsschleife (48) angeordnet und zumindest dazu konfiguriert ist, den Rezirkulationsgasstrom (FR) innerhalb der Rezirkulationsschleife in einer vorbestimmten Zirkulationsrichtung (S) im Regenerationsmodus anzutreiben;
- eine erste Adsorptionsvorrichtung (60), die im Behandlungsabschnitt (46) des Hauptpfads stromabwärts des Gebläses (50) in Bezug zur vorbestimmten Zirkulationsrichtung (S) angeordnet und dazu eingerichtet ist, vorbestimmte Substanzen im Behandlungsmodus zu adsorbieren, die möglicherweise in dem Hauptgasstrom (FT) vorhanden sind;
- einen Ozongenerator (70), der in dem Behandlungsabschnitt (46) des Hauptpfads stromabwärts des Gebläses (50) in Bezug zur vorbestimmten Zirkulationsrichtung (S) angeordnet und dazu eingerichtet ist, Ozon im Regenerationsmodus zu erzeugen; und
- eine Umschaltvorrichtung, die dazu konfiguriert ist, im Behandlungsmodus den Eingang (22) und den Ausgang (24) offen zu halten, die Zirkulation des Hauptgasstroms (FT) durch den sekundären Pfad (40) oder jeden sekundären Pfad (40, 202, 302) zu verhindern und den Ozongenerator (70) ausgeschaltet zu halten, und im Regenerationsmodus den Eingang (22) und den Ausgang (24) geschlossen zu halten, die Zirkulation des Rezirkulationsgasstroms (FR) im sekundären Pfad (40) oder in jedem sekundären Pfad (40, 202, 302) zu ermöglichen und den Ozongenerator (70) im Betrieb zu halten;
**dadurch gekennzeichnet, dass** es ferner eine zweite Adsorptionsvorrichtung (80) umfasst, die dazu eingerichtet ist, die vordefinierten Substanzen zu adsorbieren, und im Behandlungsabschnitt (46) des Hauptpfads stromabwärts des Gebläses (50) in Bezug zur vorbestimmten Zirkulationsrichtung derart angeordnet ist, dass die erste und die zweite Adsorptionsvorrichtung (60, 80) in Bezug zur vorbestimmten Zirkulationsrichtung (S) jeweils stromaufwärts und stromabwärts des Ozongenerators (70) angeordnet sind.

2. System nach Anspruch 1, wobei der sekundäre Pfad (40) einen aktiven Abschnitt (84) umfasst, der dazu konfiguriert ist, die Zusammensetzung des Rezirkulationsgasstroms (FR) im Regenerationsmodus zu modifizieren.

3. System nach Anspruch 2, wobei der aktive Abschnitt eine Behandlungsanordnung (100) umfasst, die eine Katalysatorvorrichtung (92) umfasst, die ausgelegt ist, mindestens eine Reaktion unter Oxidationsreaktionen der vordefinierten Substanzen zu katalysieren, und wobei das Gebläse (50), die erste Adsorptionsvorrichtung (60), der Ozongenerator (70), die zweite Adsorptionsvorrichtung (80) und die Behandlungsanordnung (100) in dieser Reihenfolge in Bezug zur vorbestimmten Zirkulationsrichtung (S) angeordnet sind.

4. System nach Anspruch 3, wobei die Behandlungsanordnung (100) ferner eine Ozonbehandlungsvorrichtung (94) umfasst, die dazu eingerichtet ist, Ozon einzufangen oder zu zersetzen, und stromabwärts der Katalysatorvorrichtung (92) in Bezug zur vorbestimmten Zirkulationsrichtung (S) angeordnet ist.

5. System nach Anspruch 4, wobei die Behandlungsanordnung (100) ferner eine Einfangvorrichtung (96) umfasst, die dazu eingerichtet ist, mindestens ein Produkt mindestens einer Reaktion unter Oxidationsreaktionen der vordefinierten Substanzen einzufangen, und stromabwärts der Ozonbehandlungsvorrichtung (94) in Bezug zur vorbestimmten Zirkulationsrichtung (S) angeordnet ist.

6. System nach einem der Ansprüche 2 bis 5, wobei der aktive Abschnitt (84) eine Stickstoffoxid-Behandlungsvorrichtung (90) umfasst, die dazu eingerichtet ist, Stickstoffoxide (NOx) einzufangen.

7. System nach der Kombination der Ansprüche 3 und 6, wobei die Stickstoffoxid-Behandlungsvorrichtung (90) zwischen der zweiten Adsorptionsvorrichtung (80) und der Behandlungsanordnung (100) angeordnet ist.

8. System nach einem der Ansprüche 2 bis 5, wobei der sekundäre Pfad ein erster sekundärer Pfad (40) ist, der aktive Abschnitt ein erster aktiver Abschnitt (84) ist und die Rezirkulationsschleife eine erste Rezirkulationsschleife (48) ist, wobei das System ferner einen zweiten sekundären Pfad (202) umfasst, der den ersten aktiven Abschnitt (84) nicht umfasst und zwei gegenüberliegende Enden aufweist, die jeweils stromaufwärts und stromabwärts des Behandlungsabschnitts (46) mit dem Hauptpfad (40) derart verbunden sind, dass der zweite sekundäre Pfad (202) und mindestens der Behandlungsabschnitt (46) des Hauptpfads eine zweite Rezirkulationsschleife (206) bilden, die eine Schleifenzirkulation des Rezirkulationsgasstroms (FR) im Behandlungsabschnitt (46) des Hauptpfads im Regenerationsmodus gestattet, wobei der zweite sekundäre Pfad (202) einen zweiten aktiven Abschnitt (204) umfasst, der nicht im ersten sekundären Pfad (40) enthalten ist und mindestens einen Partikelfilter (212) umfasst, wobei die Umschaltvorrichtung dazu konfiguriert ist, aufeinanderfolgende Rezirkulationsphasen durchzuführen, in denen der Rezirkulationsgasstrom (FR) im Regenerationsmodus jeweils in der ersten Rezirkulationsschleife (48) und in der zweiten Rezirkulationsschleife (206) zirkuliert.

9. System nach Anspruch 8, ferner umfassend einen dritten sekundären Pfad (302), der die jeweiligen aktiven Abschnitte (84, 204) des ersten sekundären Pfad (40) und des zweiten sekundären Pfad (202) nicht umfasst und zwei gegenüberliegende Enden aufweist, die jeweils stromaufwärts und stromabwärts des Behandlungsabschnitts (46) mit dem Hauptpfad (40) derart verbunden sind, dass der dritte sekundäre Pfad (302) und mindestens der Behandlungsabschnitt (46) des Hauptpfads eine dritte Rezirkulationsschleife (306) bilden, die eine Schleifenzirkulation des Rezirkulationsgasstroms (FR) im Behandlungsabschnitt (46) des Hauptpfads im Regenerationsmodus gestattet, wobei der dritte sekundäre Pfad (302) einen dritten aktiven Abschnitt (304) umfasst, der weder im ersten sekundären Pfad (40) noch im zweiten sekundären Pfad (202) enthalten ist und dazu konfiguriert ist, die Zusammensetzung des Rezirkulationsgasstroms (FR) im Regenerationsmodus zu modifizieren, wobei die Umschaltvorrichtung dazu konfiguriert ist, aufeinanderfolgende Rezirkulationsphasen durchzuführen, in denen der Rezirkulationsgasstrom (FR) im Regenerationsmodus jeweils in der ersten Rezirkulationsschleife (48), in der dritten Rezirkulationsschleife (306) und in der zweiten Rezirkulationsschleife (206) zirkuliert.

10. System nach Anspruch 9, wobei der dritte aktive Abschnitt (304) eine Stickstoffoxid-Behandlungsvorrichtung (312) umfasst, die eingerichtet ist, um Stickstoffoxide (NOx) einzufangen.

11. System nach einem der Ansprüche 1 bis 10, wobei das Gebläse (50) im Behandlungsabschnitt (46) des Hauptpfads untergebracht und dazu konfiguriert ist, den Hauptgasstrom (FT) innerhalb des Hauptpfads zwischen dem Eingang (22) bis zum Ausgang (24) im Behandlungsmodus anzutreiben.

12. Verfahren zum Behandeln von Gas mittels eines Systems (10, 200, 300) nach einem der Ansprüche 1 bis 11, das alternativ Folgendes umfasst:
- Umsetzen des Systems im Behandlungsmodus, wobei die Umschaltvorrichtung den Eingang (22) und den Ausgang (24) offen hält, die Zirkulation des Hauptgasstroms (FT) durch den sekundären Pfad (40) oder jeden sekundären Pfad (40, 202, 302) verhindert und den Ozongenerator (70) ausgeschaltet hält, wodurch der Hauptgasstrom (FT) durch den Eingang (22) eingelassen wird und dann durch die erste und die zweite Adsorptionsvorrichtung (60, 80) passiert, wobei alle oder ein Teil der vordefinierten Substanzen, die möglicherweise im Hauptgasstrom vorhanden sind, durch Adsorption zurückgehalten werden und dann der Hauptgasstrom, in dem die möglichen vordefinierten Substanzen angereichert sind, durch den Ausgang (24) ausgestoßen wird; und
- Umsetzen des Systems im Regenerationsmodus, wobei die Umschaltvorrichtung den Eingang (22) und den Ausgang (24) geschlossen hält, die Zirkulation des Rezirkulationsgasstroms (FR) im sekundären Pfad (40) oder in jedem sekundären Pfad (40, 202, 302) ermöglicht und den Ozongenerator (70) im Betrieb hält, wodurch:
a) der Rezirkulationsgasstrom (FR), der durch das Gebläse (50) in der Rezirkulationsschleife (48) angetrieben wird, das vom Ozongenerator (70) erzeugte Ozon aufnimmt und das Ozon und mögliche durch das Ozon induzierte aktive Arten in die zweite Adsorptionsvorrichtung (80) einbringt, in der mögliche zuvor von der zweiten Adsorptionsvorrichtung adsorbierte vordefinierte Substanzen bei Kontakt mit dem Ozon und den möglichen durch Ozon induzierten aktiven Arten oxidiert und desorbiert werden,
b) die erste Adsorptionsvorrichtung (60) das Ozon und die möglichen durch Ozon induzierten aktiven Arten aufnimmt und in den Rezirkulationsgasstrom (FR) zurückdiffundiert, wodurch die zuvor von der ersten Adsorptionsvorrichtung (60) adsorbierten vordefinierten Substanzen bei Kontakt mit dem Ozon und den möglichen durch Ozon induzierten aktiven Arten oxidiert und desorbiert werden.

## Claims

1. Gas treatment system (10, 200, 300) capable of operating alternately in a treatment mode and in a regeneration mode, comprising:
- an inlet (22) and an outlet (24), and a main path (20) connecting the inlet to the outlet and comprising a treatment portion (46) so as to enable the circulation of a main gas stream (FT) from the inlet to the outlet, passing via the treatment portion, in the treatment mode;
- at least one secondary path (40) having two opposite ends (42, 44) connected to the main path (20), respectively upstream and downstream of the treatment portion (46), so that the secondary path (40) and at least the treatment portion (46) of the main path form a recirculation loop (48) enabling closed-loop circulation of a recirculation gas stream (FR) in the treatment portion (46) of the main path, in the regeneration mode;
- a fan (50) disposed in the recirculation loop (48) and configured at least to propel the recirculation gas stream (FR) within the recirculation loop in a predetermined circulation direction (S), in the regeneration mode;
- a first adsorption device (60) disposed in the treatment portion (46) of the main path, downstream of the fan (50) with respect to the predetermined circulation direction (S), and capable of adsorbing predefined substances potentially present in the main gas stream (FT), in the treatment mode;
- an ozone generator (70) disposed in the treatment portion (46) of the main path, downstream of the fan (50) with respect to the predetermined circulation direction (S), and capable of generating ozone in the regeneration mode; and
- a switching device configured to keep the inlet (22) and the outlet (24) open, prevent the circulation of the main gas stream (FT) via the secondary path (40) or each secondary path (40, 202, 302), and keep the ozone generator (70) stopped, in the treatment mode, and to keep the inlet (22) and the outlet (24) closed, allow the circulation of the recirculation gas stream (FR) in the secondary path (40) or in each secondary path (40, 202, 302), and keep the ozone generator (70) in operation, in the regeneration mode;
**characterised in that** it further comprises a second adsorption device (80) capable of adsorbing the predefined substances, disposed in the treatment portion (46) of the main path, downstream of the fan (50) with respect to the predetermined circulation direction, and such that the first and second adsorption devices (60, 80) are respectively disposed upstream and downstream of the ozone generator (70) with respect to the predetermined direction of circulation (S).

2. System according to claim 1, wherein the secondary path (40) comprises an active portion (84) configured to modify the composition of the recirculation gas stream (FR), in the regeneration mode.

3. System according to claim 2, wherein the active portion comprises a treatment assembly (100) comprising a catalysis device (92) suitable for catalysing at least one reaction among oxidation reactions of the predefined substances, and wherein the fan (50), the first adsorption device (60), the ozone generator (70), the second adsorption device (80) and the treatment assembly (100) are arranged in that order with respect to the predetermined direction of circulation (S).

4. System according to claim 3, wherein the treatment assembly (100) further comprises an ozone treatment device (94) suitable for trapping or breaking down ozone, and arranged downstream of the catalyst device (92) with respect to the predetermined direction of circulation (S).

5. System according to claim 4, wherein the treatment assembly (100) further comprises a trapping device (96) suitable for trapping at least one product of at least one reaction among the oxidation reactions of the predefined substances, and arranged downstream of the ozone treatment device (94) with respect to the predetermined direction of circulation (S).

6. System as claimed in any one of claims 2 to 5, wherein the active portion (84) comprises a nitrogen oxides treatment device (90), suitable for trapping nitrogen oxides (NOx).

7. System according to the combination of claims 3 and 6, wherein the nitrogen oxides treatment device (90) is arranged between the second adsorption device (80) and the treatment assembly (100).

8. System according to any one of claims 2 to 5, wherein the secondary path is a first secondary path (40), the active portion is a first active portion (84), and the recirculation loop is a first recirculation loop (48), the system further comprising a second secondary path (202) not comprising the first active portion (84) and having two opposite ends connected to the main path (40), respectively upstream and downstream of the treatment portion (46), so that the second secondary path (202) and at least the treatment portion (46) of the main path form a second recirculation loop (206) allowing a closed-loop circulation of the recirculation gas stream (FR) in the treatment portion (46) of the main path, in the regeneration mode, the second secondary path (202) comprising a second active portion (204) not included in the first secondary path (40) and comprising at least one particle filter (212), the switching device being configured to cause a succession of recirculation phases in which the recirculation gas stream (FR) circulates respectively in the first recirculation loop (48) and in the second recirculation loop (206), in the regeneration mode.

9. System according to claim 8 further comprising a third secondary path (302) not comprising the respective active portions (84, 204) of the first secondary path (40) and of the second secondary path (202) and having two opposite ends connected to the main path (40), respectively upstream and downstream of the treatment portion (46), so that the third secondary path (302) and at least the treatment portion (46) of the main path form a third recirculation loop (306) allowing a closed-loop circulation of the recirculation gas stream (FR) in the treatment portion (46) of the main path, in the regeneration mode, the third secondary path (302) comprising a third active portion (304) not included in the first secondary path (40) or in the second secondary path (202) and configured to modify the composition of the recirculation gas stream (FR), in the regeneration mode, the switching device being configured to cause a succession of recirculation phases in which the recirculation gas stream (FR) circulates respectively in the first recirculation loop (48), in the third recirculation loop (306) and in the second recirculation loop (206), in the regeneration mode.

10. System according to claim 9, wherein the third active portion (304) comprises a nitrogen oxides treatment device (312), suitable for trapping nitrogen oxides (NOx).

11. System according to any one of claims 1 to 10, wherein the fan (50) is housed in the treatment portion (46) of the main path and is configured to propel the main gas stream (FT) within the main path from the inlet (22) to the outlet (24), in the treatment mode.

12. Method for gas treatment by means of a system (10, 200, 300) according to any one of claims 1 to 11, comprising, alternatively:
- using the system in the treatment mode, in which the switching device keeps the inlet (22) and the outlet (24) open, prevents the circulation of the main gas stream (FT) via the secondary path (40) or each secondary path (40, 202, 302), and keeps the ozone generator (70) stopped, whereby the main gas stream (FT) is admitted by the inlet (22), then passes into the first and second adsorption devices (60, 80), wherein all or part of the predefined substances potentially present in the main gas stream are retained by adsorption, then the main gas stream, depleted of any predefined substances, is discharged via the outlet (24); and
- using the system in regeneration mode, in which the switching device keeps the inlet (22) and the outlet (24) closed, allows the circulation of the recirculation gas stream (FR) in the secondary path (40) or in each secondary path (40, 202, 302), and keeps the ozone generator (70) operating, whereby:
a) the recirculation gas stream (FR), driven by the fan (50) in the recirculation loop (48), charges with ozone produced by the ozone generator (70) and carries away the ozone and any active species induced by the ozone, into the second adsorption device (80), wherein any predefined substances previously adsorbed by the second adsorption device are oxidised and are desorbed on contact with the ozone and any other active species induced by the ozone,
b) the first adsorption device (60) receives the ozone, and any active species induced by the ozone, potentially backscattering into the recirculation gas stream (FR), whereby any predefined substances previously adsorbed by the first adsorption device (60) are oxidised and are desorbed on contact with the ozone and any active species induced by the ozone.
